# EUROPÄISCHE PATENTSCHRIFT

(11) **EP 2 512 324 B1**
(45) Veröffentlichungstag und Bekanntmachung des Hinweises auf die Patenterteilung: **14.09.2016**
(21) Anmeldenummer: 10809230.5
(22) Anmeldetag: 16.12.2010
(51) Int. Cl.: G01R 33/09, A61B 3/113, A61B 5/00, A61F 2/16

(54) **IMPLANTIERBARES SYSTEM ZUR BESTIMMUNG DES AKKOMMODATIONSBEDARFS**
IMPLANTABLE SYSTEM FOR DETERMINING ACCOMMODATION NEED
SYSTÈME IMPLANTABLE SERVANT À DÉTERMINER LA DEMANDE EN ACCOMMODATION

(30) Priorität: 18.12.2009 DE 102009059229
(43) Veröffentlichungstag der Anmeldung: 24.10.2012
(73) Patentinhaber: Karlsruher Institut für Technologie, 76131 Karlsruhe (DE)
(72) Erfinder: RHEINSCHMITT, Liane, 76297 Stutensee (DE); RITTER, Fabian, 74391 Erligheim (DE); NAGEL, Jörg, 67071 Ludwigshafen (DE); GENGENBACH, Uli, 75196 Remchingen (DE); MARTIN, Thomas, 76139 Karlsruhe (DE); BRETTHAUER, Georg, 76139 Karlsruhe (DE); GUTHOFF, Rudolf, 18119 Rostock (DE)
(74) Vertreter: Fitzner, Uwe
(86) Internationale Anmeldenummer: PCT/EP2010/069946
(87) Internationale Veröffentlichungsnummer: WO 2011/080107

(56) Entgegenhaltungen:
- WO-A1-2006/034336
- DE-A1-102007 008 374
- US-A1- 2002 091 421
- US-B1- 6 579 235
- SMITHA SHANKAR, MICHAEL AUSTIN: "MEMS Glaucoma Monitoring System", SPIE, PO BOX 10 BELLINGHAM WA 98227-0010 USA, Bd. 6528, April 2007 (2007-04), Seiten 65280W-1-65280W-10, XP040238714,
- SMITHA SHANKAR, J.P. CHAFFEY: "Development of a MEMS device to monitor Glaucoma", SPIE, PO BOX 10 BELLINGHAM WA 98227-0010 USA, Bd. 5649, Februar 2005 (2005-02), Seiten 648-658, XP040197774,

## Beschreibung

Gegenstand der Erfindung ist ein implantierbares System zur Bestimmung des Akkommodationsbedarfs in einem künstlichen Akkommodationssystem und dessen Verwendung zur Behandlung des Verlusts der Akkomodationsfähigkeit.

Das menschliche Auge ist ein optisches System, das mit Hilfe mehrerer lichtbrechender Grenzflächen Objekte scharf auf der Netzhaut (retina) abbildet. Hierbei passieren die Lichtwellen die Hornhaut (cornea), das Kammerwasser in der Vorderkammer (camera anterior bulbi), die Linse (lens crystallina) und den Glaskörper in der Hinterkammer (camera vitrea bulbi), die alle unterschiedliche Brechungsindizes aufweisen. Ändert sich die Gegenstandsweite des betrachteten Objektes, ist es für eine Abbildung mit gleich bleibender Schärfe auf der Netzhaut notwendig, dass sich das Abbildungsverhalten des optischen Systems ändert. Beim menschlichen Auge wird dies durch eine Verformung der Linse mit Hilfe des Ziliarmuskels (musculus ciliaris) realisiert, wodurch sich im Wesentlichen die Form und die Lage der Linsenvorder- und -rückseite ändern (Akkommodation). Bei einem intakten Akkommodationssystem eines jugendlichen Menschen kann so die Scheitelbrechkraft des Systems zwischen Ferneinstellung (desakkommodierter Zustand) und Naheinstellung (akkommodierter Zustand) um 14 dpt (Akkommodationsbreite) verändert werden. Dadurch können bei einem normalsichtigen (emmetropen) jugendlichen Menschen Objekte, die sich zwischen dem im Unendlichen liegenden Fernpunkt und dem sich in etwa 7 cm vor der Hornhaut liegenden Nahpunkt befinden, scharf auf der Netzhaut abgebildet werden.

Da die Fähigkeit des menschlichen Auges zur Akkommodation mit zunehmendem Alter abnimmt, sind eine Anzahl von künstlich implantierbaren Linsensystemen mit variabler Brennweite entwickelt worden.

Bei potentiell akkommodierenden Intraokularlinsen handelt es sich um Linsen oder Linsensysteme, die nach operativer Entfernung der natürlichen Linse anstelle dieser eingesetzt und vorwiegend im Kapselsack befestigt werden. Durch eine noch vorhandene, jedoch geringe Restkontraktion des Ziliarmuskels, soll über eine Haptik eine axiale Verschiebung der Linse erreicht werden.

Beispiele für die Vielzahl der Entwicklungen sind unter anderem in DE 94 22 429 U1, DE 201 11 320 U1, DE 100 62 218 A1, DE 101 39 027, WO 02/083033, DE 101 25 829 A1, US 2004/0181279A1, US 2002/0149743, US 6 120 538, US 6 120 538, DE 101 55 345 C2, US 6 096 078, US 6 638 304, US 6 638 304, WO 00/4605 und WO 00/4605 beschrieben.

Vorrichtungen zur Wiederherstellung der Akkomodationsfähigkeit sind ferner aus der DE 101 55 345 C2, US 6 638 304 B2, WO 03/017873 A1 und US 43,73218 bekannt.

Ferner gibt es zahlreiche wissenschaftliche Veröffentlichungen zu dem Thema Akkommodationsfähigkeit von Linsensystemen. Beispielhaft sei auch auf folgende Veröffentlichung verwiesen:
Schneider, H.; Stachs, O.; Guthoff, R.: Evidenzbasierte Betrachtungen zu akkommodativen Kunstlinsen. 102. Jahrestagung der Deutschen Ophthalmologischen Gesellschaft (Berlin, Germany, September 23rd-26th 2004) (2004); Kammann, J.; Dornbach, G.: Empirical results regarding accommodative lenses. In: Current Aspects of Human Accommodation. Hrsg.: Guthoff, R.; Ludwig, K. Kaden Verlag Heidelberg (2001) 163-170, Fine, H.; Packer M.; Hoffmann R.: Technology generates IOL with amplitude of accommodation" (Ophtalmology Times Special Report, March 15th 2005) (2005), Lavin, M.: Multifocal intraocular lenses - part 1. Optometry Today 5/2001 (2001) 34-37; Lavin, M.: Multifocal intraocular lenses - part 2. Optometry Today 8/2001 (2001) 43-44; Nishi, O., Nishi, K.; Mano, C.; Ichihara, M.; Honda, T.: Controlling the capsular shape in lens refilling. Archives of Ophthalmology 115(4) (1997) 507-510; Fine, I.H.: The SmartLens- a fabulous new IOL technology. Eye World 7(10) (2002).

Das Problem der Akkommodation bis zu einem Leseabstand von ca. 30 cm wird durch den bisherigen Stand der Technik noch nicht zufriedenstellend gelöst. Grundsätzlich ist es nämlich durch die im Rahmen einer Kataraktextraktion implantierte Kunstlinse bisher nicht zufriedenstellend möglich, auf verschiedene Entfernungen zu fokussieren. Bisherige Versuche, intraokulare Strukturen, insbesondere die Ziliarmuskelaktivität zur mechanischen Brechkraftänderung implantierbarer Systeme zu nutzen, sind aus biologischen Gründen bisher nicht gelungen und dies ist auch mittelfristig nicht zu erwarten.

Aus dem Stand der Technik sind Lösungen zur intrakorporalen Bestimmung des Akkommodationsbedarfs über die Augapfelorierntierung des Augenpaares bekannt. Diese beschränken sich aber auf die Detektion der Kontraktion der äußeren Augenmuskeln, entweder durch Potentialmessung (z.B. US 6,638304) oder durch Messung der Andruckdifferenz zwischen Augapfel und zwei horizontalen Bulbusmuskeln an unterschiedlichen Augen (WO 2004/004605A1).

Die Erzeugung von starken, wechselnden elektromagnetischen Feldern sowie die Detektion der Bewegung kleiner Spulen setzen Geräte voraus, die aufgrund ihrer Masse und ihres Volumens für eine Implantation nicht geeignet sind. Die Messung der Kontraktion der Bulbusmuskeln über das Muskelpotential oder des Andrucks setzt eine Informationsverbindung zwischen Sensor und dem optisch aktiven Implantat im Kapselsack voraus. Eine Kabelverbindung würde den operativen Aufwand enorm erhöhen. Eine drahtlose Informationsübertragung setzt ein aktives System am Muskel voraus, welches ebenfalls über eine Energieversorgung verfügen müsste. Zusätzlich besteht bei der Verwendung von Elektroden das Problem von möglichen Gewebeänderungen im Elektrodenbereich. Daher sind beide Lösungen nicht praktikabel.

Aus der DE 10 2005 038 542 A1 ist ein künstliches Akkomodationssystem bekannt, welches wenigstens ein optisches System, wenigstens ein Informationserfassungssystem zwecks Erfassung der körpereigenen Steuersignale für die Pupillenweite oder Augenmotorik oder Akkomodation oder Kombination der genannten Steuersignale, wenigstens ein Informationsverarbeitungssystem zwecks Erzeugung eines Stellsignals für das optische System aus den erfassten körpereigenen Signalen, wenigstens ein Energieversorgungssystem und wenigstens ein Befestigungssystem umfasst.

Aus der DE 10 2007 008 374 B1 ist die Weiterentwicklung eines solchen Systems bekannt. Dies weist in beiden Augen je ein Mittel zur Erfassung der Augapfelorientierung des jeweiligen Augapfels auf der Basis der Messung des externen Magnetfeldes auf, wobei die Mittel zur Erfassung der Augapfelorientierung fest mit dem jeweilig zugehörigen Augapfel verbunden sind, oder in diesen eingesetzt sind. Ferner sind Übertragungsmittel für den gegenseitigen Informationsaustausch zwischen den Mitteln zur Erfassung der Augapfelorientierung des jeweiligen Augapfel vorgesehen.

Das Implantat gemäß der DE 10 2007 008 374 B1 beinhaltet eine aktive Optik, die sich an die Gegenstandsweiten verschiedener vom Patienten fokussierter Objekte anpasst. Dazu wird zunächst der Abstand zum Objekt mit Hilfe eines Sensorsystems bestimmt. Diese Information wird in einer Steuereinheit ausgewertet und an einen Aktor weitergeleitet, der die aktive Optik anpasst.

Die Nutzung des Erdmagnetfeldes zur Bestimmung des Vergenzwinkels ist mit verschiedenen Nachteilen verbunden. Die Richtung und der Inklinationswinkel des Erdmagnetwinkels relativ zum Implantat sind von der Position des Patienten auf der Erde abhängig. Der Inklinationswinkel beträgt z.B. in Baden-Württemberg 64 Grad, während am Äquator das Erdmagnetfeld parallel zur Erdoberfläche verläuft. Somit kann die Sensorlage im Implantat nicht optimiert ausgerichtet werden. Zum anderen wird das Magnetfeld in Gebäuden stark abgelenkt und kann so stark variieren, dass Inhomogenitäten zwischen den beiden Augenimplantaten auftreten, die fälschlicherweise als Messwert interpretiert werden. Störeinflüsse können z.B. auch stählerne Tischbeine auf die Erfassung des Vergenzwinkels haben. Gleiches gilt z. B. für Einflüsse durch Lautsprechermagnete, die in Mobiltelefonen oder Kopfhörern verbaut werden.

Der Magnetfeldsensor ist nicht auf eine von vielen Faktoren abhängige ideale Lage optimierbar. Daher sollte er stets horizontal ausgerichtet sein. Für die Nutzung des Bauraums im künstlichen Akkommodationssystem ist dies ein Nachteil. Die elektronischen Komponenten sollten nach Möglichkeit in einer Ebene angeordnet sein, da die Positionierung von eckigen Bauteilen in einem runden Gehäuse sonst zu nicht bzw. schwer nutzbaren Bereichen führt. Bei der Anordnung des Magnetfeldsensors auf einer Leiterkarte muss zudem der Biegeradius bzw. der Bauraum für die Steckkontakte berücksichtigt werden. Die Integration des Magnetfeldsensors in einem großflächigen ASIC (Anwendung spezifische integrierte Schaltung) ist zum derzeitigen Stand der Technik nicht möglich.

Alternativ zur Messung des Erdmagnetfeldes wird in der DE 10 2007 008 374 B4 die Verwendung eines kopffesten Magnetfeldes vorgeschlagen. Dafür ist aber eine zusätzliche Implantation nötig. Dies bedeutet ein zusätzliches Operationsrisiko für den Patienten. Zudem ist die Körperverträglichkeit des ausgesendeten Feldes im Kopfbereich noch nicht ausreichend untersucht.

Beim Einsatz von Magnetfeldmessungen gibt es ferner einen Kopfneigungswinkel, bei dem der Vergenzwinkel nicht bestimmt werden kann, da die Feldlinien parallel zur Drehachse verlaufen und somit keine Änderung des Magnetfeldes während der Vergenz messbar ist. Hierzu wird in der DE 10 2007 008 374 B4 keine Lösung angegeben. In diesem Bereich ist demnach keine Bestimmung des Akkomodationsbedarfs durch das System möglich. Da aufgrund der Ausrichtung des Erdmagnetfeldes in einigen Regionen der Erde bevorzugte Sehwinkel mit dem nicht erfassbaren Bereich zusammenfallen, ist dieses System gemäß der DE 10 2007 008 374 B4 zumindest nicht in alleiniger Anwendung für künstliche Akkommodationssysteme geeignet.

Die vorliegende Erfindung hat sich nunmehr die Aufgabe gestellt, die Nachteile des Standes der Technik, insbesondere der künstlichen Akkommodationssysteme, welche den Vergenzwinkel unter Messung des externen Magnetfeldes ermitteln, zu vermeiden.

Gegenstand der Erfindung ist demgemäß ein implantierbares System zur Bestimmung des Akkommodationsbedarfs in einem künstlichen Akkommodationssystem durch Messung der Augapfelorientierung umfassend
a) wenigstens ein optisches System,
b) wenigstens ein Informationserfassungssystem mit Mitteln zur Erfassung der Augapfelorierntierung beider Augäpfel als körpereigenes Steuersignal für den Akkommodationsbedarf,
c) wenigstens ein Informationsverarbeitungssystem zwecks Erzeugung eines Stellsignals für das optische System aus den erfassten körpereigenen Steuersignalen
d) wenigstens ein Energieversorgungssystem und
e) wenigstens ein Befestigungssystem,
wobei Übertragungsmittel für den gegenseitigen Informationsaustausch zwischen den Mitteln zur Erfassung der Augapfelorientierung des jeweiligen Augapfels vorgesehen sind.
Gelöst wird die erfindungsgemäße Aufgabe dadurch, dass das System in beiden Augen je ein Mittel zur Erfassung der Augapfelorientierung des jeweiligen Augapfels auf der Basis der Messung des externen Beschleunigungsfeldes aufweist, wobei die Mittel zur Erfassung der Augapfelorientierung fest mit den jeweiligen zugehörigen Augapfel verbunden sind oder in diesen eingesetzt sind.

Das künstliche Akkommodationssystem ist der technische Teil eines Regelungssystems (geschlossener Regelkreis), welches als künstliches System die Funktion der natürlichen verformbaren Augenlinse und des Ziliarmuskels eines Patienten substituiert. Der biologische Teil besteht im Wesentlichen aus: der Hornhaut, dem Kammerwasser und dem Glaskörper als Bestandteile des dioptrischen Apparates, der Netzhaut als natürlichem Sensorarray und dem Gehirn als natürliche Informationsverarbeitungseinheit, die Steuersignale erzeugt, die Informationen über den Akkommodationsbedarf enthalten.

Das künstliche Akkommodationssystem umfasst ein optisches System, mit einer verstellbaren Brennweite und/oder anderen optischen Eigenschaften. Dieses bildet einen neu eingebrachten Bestandteil des dioptrischen Apparates des Patienten. Es umfasst ein Informationserfassungssystem, das Mittel zur Messung des Beschleunigungsfeldes und ggf. in Kombination mit der Größe des Magnetfeldes aufweist. Auf Basis dieser Messungen wird der Akkommodationsbedarf durch ein Informationsverarbeitungs-system ermittelt und es werden Stellsignale zum Ansteuern des optischen Systems generiert. Das System wird über ein geeignetes Energieversorgungssystem gespeist und ist über ein Befestigungssystem im Patientenauge fixiert.

Die Subsysteme a) bis e) des künstlichen Akkommodationssystems sind in der DE 10 2005 038 542 A1 und DE 10 2007 008 374 B4 beschrieben. Diese Systeme sind zu einem bzw. in mehreren Regelkreisen verschaltet. Das optische System, das Informationserfassungssystem, das Informationsverarbeitungssystem, das Energieversorgungssystem und das Befestigungssystem sind vorzugsweise zu einem Implantat zusammengefasst, welches zur Wiederherstellung der Akkommodationsfähigkeit des tierischen oder menschlichen Auges in dieses mittels des Befestigungssystems einsetzbar ist. Hierbei ist das optische System im Strahlengang des Auges angeordnet und bildet mit diesem zusammen den dioptrischen Apparat des Auges. In gleicher Weise sind vorzugsweise das Informationserfassungssystem, das Informationsverarbeitungssystem und das Energieversorgungssystem außerhalb des Strahlengangs angeordnet. Das Informationserfassungssystem kann sich auf mehrere Implantate (z. B. im linken und rechten Augapfel und im Oberkiefer) verteilen. Das Energieversorgungssystem kann vorzugsweise drahtlos mit einem externen System in Verbindung stehen.

Das optische System, bestehend aus einem oder mehreren aktiv-optischen Elementen und / oder einer oder mehreren von Aktoren axial verschieblichen starren Linsen (=passiv-optisches Element), hat die Aufgabe, das Abbildungsverhalten im Strahlengang zu beeinflussen. Es muss im sichtbaren Wellenlängenbereich transparent sein und muss die Lage und / oder die Form mindestens einer seiner lichtbrechenden Grenzflächen zeitlich ändern können, um die Scheitelbrechkraft des dioptrischen Apparates zu verändern. Die aktorische Komponente besteht dabei aus Energiestellern und Energiewandlern (Grote / Feldhusen (Hrsg.): Dubbel - Taschenbuch für den Maschinenbau. 21. Auflage. Springer Verlag Berlin Heidelberg New York (2005)), welche unter Einwirkung von Stellsignalen einer informationsverarbeitenden Einrichtung Kräfte realisiert, die dann in Bewegung umgesetzt werden können.

Im Falle eines passiv-optischen Elementes werden eine oder mehrere starre Linsen von einem Aktor axial im Strahlengang verschoben. Dieses Wirkprinzip wird standardgemäß in technischen Produkten zur Fokussierung eingesetzt. DE4300840A1 beschreibt z.B. ein Varioobjektiv für Kompaktkameras, das aus zwei Linsengruppen besteht, deren Abstand relativ zueinander variiert werden kann, um eine Brennweitenverstellung durchzuführen.

Verschiedene Mechanismen können zur Erfüllung der oben beschriebenen Aufgabe eines aktiv-optischen Elements zum Einsatz kommen. Dabei ist zwischen einer Änderung der Brechungsindexverteilung und einer Krümmungsänderung einer zwei Medien unterschiedlicher Brechungsindizes trennenden Grenzfläche zu unterscheiden. Diese Veränderungen können durch verschiedene physikalische Wirkungsprinzipien realisiert werden, die im Folgenden dargestellt werden. Brechungsindexänderung durch elektrooptische Materialien: Die doppelbrechende Eigenschaft elektrooptischer Materialien kann durch elektromagnetische Felder beeinflusst werden. Dadurch kann eine definierte Brechungsindexverteilung eingestellt werden, die eine gezielte Beeinflussung des Abbildungsverhaltens in einer Polarisationsebene des Lichts ermöglicht. Diese kann neben einer gezielten Änderung der Fokuslage auch die Korrektur von Abbildungsfehlern höherer Ordnung (z.B. Astigmatismen, sphärische Aberration, Koma) umfassen. Um beide zueinander senkrechten Polarisationsebenen gleichermaßen zu beeinflussen, ist eine rechtwinklig gekreuzte Hintereinanderanordnung zweier derartiger Systeme notwendig. In US6619799 wird die Verwendung eines derartigen aktiv-optischen Elementes in einem Brillengestell beschrieben. Dabei ist die elektrooptische Schicht von zwei transparenten Elektrodenflächen umfasst, zwischen denen eine elektrische Spannung angelegt werden kann, um das radiale Brechungsindexprofil zu verändern. Ein gewünschtes Brechungsindexprofil kann entweder durch Amplituden- und Frequenzmodulation der Steuerspannung oder durch Aufteilung der Elektroden in mehrere Bereiche, die mit jeweils unterschiedlichen Spannungen versorgt werden, erreicht werden.

Brechungsindexänderung durch Dichteänderung eines kompressiblen Fluids: Der Brechungsindex eines kompressiblen Fluids (z.B. eines Gases oder Gasgemisches) ist von der Dichte abhängig. Diese Abhängigkeit wird durch die Gladstone-Dale-Konstante beschrieben. Werden in einer gasgefüllten Kammer, die ein oder mehrere gekrümmte Begrenzungsflächen aufweist, der Druck und / oder die Temperatur geändert, ändert sich demnach auch das Abbildungsverhalten des optischen Systems. US4732458 beschreibt z.B. eine derartige Anordnung für ein Mehrlinsenelement, dessen Brechkraft kontinuierlich verändert werden kann. Die Druckerhöhung in der starren gasgefüllten Kammer wird durch einen in einem Zylinder geführten verschieblichen Kolben, der außerhalb der optischen Achse angeordnet ist, realisiert.

Geometrieänderung durch äußere Krafteinwirkung auf einen elastischen Festkörper: Ein elastischer Festkörper, dessen Brechungsindex sich von dem der Umgebung unterscheidet, kann durch Einwirkung äußerer Kräfte so verformt werden, dass sich die Krümmung seiner lichtbrechenden Oberflächen ändert und dadurch das optische Abbildungsverhalten beeinflusst wird. US6493151 beschreibt z.B. eine Anordnung für einen derartig verformbaren homogen oder inhomogen aufgebauten Festkörper, auf den durch einen in seinem Durchmesser veränderbaren Ring radiale Kräfte übertragen werden können. Die Durchmesseränderung des Rings kann thermisch, oder durch magnetische / elektrische Felder erfolgen. DE4345070 beschreibt beispielsweise eine Anordnung für einen verformbaren hüllenförmigen Festkörper, der mit einer lichtdurchlässigen Flüssigkeit gefüllt ist und dessen lichtbrechende Oberflächen über einen ringförmigen Fluidaktor hydraulisch oder pneumatisch verformt werden. DE10244312 nennt als Anwendungsbeispiel für einen Aktor aus Buckypaper (papierartiges Netzwerk von Kohlenstoffnanoröhren) die Änderung der Brechkraft einer künstlichen, in den Augapfel implantierten deformierbaren Linse.

Geometrieänderung durch Benetzungswinkelbeeinflussung (Electrowetting): Zwei ineinander nicht mischbare Fluide annähernd gleicher Dichte, die sich in ihren Brechungsindizes unterscheiden, bilden eine sphärisch gekrümmte oder plane Grenzfläche (Meniskus). Wird das eine, elektrisch leitfähige Fluid, in Kontakt mit einer Elektrode gebracht und gegenüber einer zweiten, von den beiden Fluiden durch eine isolierende Schicht (Dielektrikum) getrennte Elektrode eine Potentialdifferenz angelegt, so lässt sich der Benetzungswinkel und somit die Krümmung des Meniskus durch den sog. Elektrowetting-Effekt ändern. Da der Meniskus zwei Medien unterschiedlichen Brechungsindex trennt, wird das optische Abbildungsverhalten verändert. WO99/18456 beschreibt eine axiale Anordnung von leitfähigem Fluid, transparentem Dielektrikum und transparenter Elektrode im Strahlengang und Maßnahmen zur radialen Zentrierung des Tropfens in der optischen Achse. WO03/069380 beschreibt eine Anordnung, bei der die mit einem Dielektrikum beschichtete Elektrode zylindrisch um die optische Achse angeordnet ist. In der optischen Achse befinden sich axial hintereinander angeordnet das elektrischleitfähige Fluid und das isolierende Fluid, sowie der die beiden trennende Meniskus.

Geometrieänderung durch Druckänderung eines Fluides: Wird in einer fluidbefüllten Kammer, die eine oder mehrere deformierbare Begrenzungsflächen aufweist, die Druckdifferenz zur Umgebung geändert, kommt es zu einer Krümmungsänderung der Begrenzungsflächen und demnach auch zur Änderung des Abbildungsverhaltens des optischen Systems. US4466706 beschreibt beispielsweise eine derartige Anordnung, wobei die Druckdifferenzänderung durch einen Verdrängungsmechanismus erreicht wird. Dabei wird durch Drehung einer sich in der zylindrischen Ummantelung befindliche Schraube Fluid verdrängt, was zur Krümmungsänderung der beiden Zylinderendflächen führt. Alternativ kann die zylindrische Ummantelung auch zweiteilig ausgeführt sein, wobei eine Verdrängungswirkung durch eine axiale Relativbewegung beider Teile erzielt werden kann.

Geometrieänderung durch Kraftentwicklung innerhalb eines intelligenten Materials: Intelligente Materialien (Smart Materials) können durch Änderungen ihrer atomaren / molekularen Struktur Kräfte entwickeln und sich dadurch verformen. Durch die Einstellung eines Grenzflächenprofils zwischen intelligentem Material und Umgebung lässt sich demzufolge ebenfalls das optische Abbildungsverhalten beeinflussen. US2004/0100704 beschreibt z.B. einen zu diesem Zwecke verwendeten Formgedächtniskunststoff, der als Phase oder Schicht innerhalb eines verformbaren Linsenkörpers eingebracht ist und die Form des Körpers unter Einwirkung von Energie lokal verändern kann. Als Anwendungsbeispiel wird die postoperative, nicht reversible Korrektur des Abbildungsverhaltens implantierter Intraokularlinsen genannt. JP01230004 beschreibt beispielsweise die Verwendung eines schwellbaren Gels und eines Lösungsmittels, die innerhalb eines deformierbaren Festkörpers schichtartig angeordnet sind. Unter Einwirkung einer Spannung kann die Löslichkeit des Lösungsmittels im schwellbaren Gel so verändert werden, dass dieses daraufhin eine Volumenänderung erfährt. Diese bewirkt eine Krümmungsänderung der lichtbrechenden Oberfläche.

Auch Kombinationen der genannten Wirkprinzipien sind möglich. Das optische System ist folglich in der Lage, die Fokuslage des dioptrischen Apparates anzupassen. Das optische System kann des Weiteren mehrere Elemente umfassen, um das optische Abbildungsverhalten im Strahlengang zu optimieren. Ein enthaltenes aktiv-optisches Element kann gegebenenfalls weitere Abbildungsfehler (monochromatische und chromatische Aberrationen) statisch oder auch dynamisch korrigieren (lokale Beeinflussung der Lichtwellenfront).

Zur Generierung der Stellsignale für die aktorische Komponente des aktiv-optischen Elementes bzw. des passiv-optischen Elementes, ist es notwendig, Informationen zu erfassen, aus denen auf die notwendige Scheitelbrechkrafterhöhung (=Akkommodationsbedarf) geschlossen werden kann.

Informationen über den Akkommodationsbedarf können aus körpereigenen Steuersignalen der Okulomotorik gewonnen werden. Unter Steuersignalen werden hierbei Informationen verstanden, die den Sollwert oder den unter Einfluss des Sollwertes und möglicher Störsignale umgesetzten Istwert einer Regelstrecke enthalten. Für die Nutzung der Steuersignale der Okulomotorik, müssen Informationen beider Augen zusammen zur Ermittlung des notwenigen Akkommodationsbedarfs herangezogen werden.

Die Okulomotorik (vor allem die horizontale Vergenzbewegung) und der Akkommodationsbedarf sind beim Binokularsehen eindeutig miteinander gekoppelt. Die Fixierlinien beider Augen richten sich durch Rotation der Augäpfel so auf ein beliebig im Raum positioniertes Fixationsobjekt aus, dass das Bild des Fixationsobjektes auf korrespondierende Netzhautstellen fällt. Dadurch wird die Informationsfusion der beiden Einzelbilder zu einem Einfachbild im Gehirn ermöglicht. Die räumliche Orientierung der Augäpfel kann durch die Rotationen der Augäpfel um die drei Raumachsen beschrieben werden. Dabei treten bei jedem Augapfel separat betrachtet Rotationen um die horizontale Achse (Nickbewegung), um die mitgedrehte vertikale Achse (Gierbewegungen) und die mitgedrehte Fixierlinie (Rollbewegungen) auf. Dementsprechend können in Bezug auf beide Augen konjugierte Augenbewegungen (Versionen = gleichsinnige, gleichgroße Bewegung der Fixierlinien oder der Netzhautmeridiane beider Augen) und disjungierte Augenbewegungen (Vergenzen = gegensinnige, gleichgroße Bewegung der Fixierlinien oder der Netzhautmeridiane beider Augen) unterschieden werden. Im Allgemeinen unterscheiden sich die Abstände des Fixationsobjektes zu den beiden mechanischen Augendrehpunkten und damit auch der Akkommodationsbedarf geringfügig (dies ist insbesondere dann der Fall, wenn sich das Fixationsobjekt nicht symmetrisch zu und nahe an den beiden Augen befindet). Die Erfassung der Steuersignale der Okulomotorik (Nervensignale oder Muskelsignale) ist extrakorporal möglich (z.B. durch die Elektromyographie der Augenmuskeln), sie wäre intrakorporal jedoch mit einem hohen Aufwand verbunden. Die Motorik der Augenbewegungen ist auch im hohen Alter hochpräzise und Abweichungen zwischen Soll- und Istwert (=Fixationsdisparität) betragen nur wenige Winkelminuten. Daher ist beim Binokularsehen in sehr guter Näherung ein Schnitt der Fixierlinien gewährleistet und es kann aus den Auswirkungen der Nerven- und Muskelsignale an die Augenmotorik, d.h. aus der Orientierung beider Augäpfel im Raum, auf den Akkommodationsbedarf des rechten, bzw. des linken Auges geschlossen werden.

Das beschriebene optisch aktive System ist in der DE 10 2007 008 374 B4 (Abschnitte [0020] - [0033]) ausführlich beschrieben und gehört mithin zum Stand der Technik. Allerdings wird nach diesem Stand der Technik - wie einleitend dargestellt - der Akkommodationsbedarf durch Einsatz von Mitteln zur Messung des Magnetfeldes aus der Stellung der beiden Augäpfel berechnet.

Die vorliegende Erfindung löst die Nachteile des Standes der Technik, welcher unter Einsatz der Messung des Einsatzes des Magnetfeldes arbeitet, durch Erfassung des Vergenzwinkels mit Hilfe des Erdbeschleunigungsfeldes. Das Sensortsystem nutzt ebenfalls die akkomodative Vergenz. Dies ist eine gegensinnige Augenbewegung, die bei der Fixierung von Objekten verschiedenen Gegenstandsweiten auftritt. Wird ein Objekt mit großem Abstand betrachtet, verlaufen die Fixierlinien der Augen nahezu parallel. Der Vergenzwinkel ist sehr klein. Je näher das Objekt sich relativ zum Betrachter befindet, umso größer wird der Winkel zwischen den Fixierlinien. Erfasst man den Vergenzwinkel, kann damit der Akkommodationsbedarf des Menschen ermittelt werden.

Um den Vergenzwinkel zu ermitteln, muss die Winkellage der beiden Implantate zueinander ermittelt werden. Dafür wird die Lage jedes einzelnen Implantates relativ zu einer feststehenden Größe ermittelt. Damit ist der Idealfall für die Erfassung der Blickrichtung der Augen senkrecht nach unten. In dieser Position befinden sich die vorzugsweise zweiachsigen Sensoren parallel zum Beschleunigungsfeld der Erde. Der Winkel im Beschleunigungsfeld ergibt sich somit aus den gemessenen Anteilen des Beschleunigungsfelds. Über eine Kommunikation zwischen den Implantaten kann der Vergenzwinkel ermittelt werden und somit der Akkommodationsbedarf berechnet werden.

Durch den erfindungsgemäßen Einsatz der Messung der Erdbeschleunigung wird es möglich, Bereiche zu erfassen, in denen bisher die Bestimmung des Akkommodationsbedarfs nicht möglich war. Zudem kann die Störanfälligkeit gegenüber der Messung des Erdmagnetfeldes deutlich reduziert werden. Hochfrequente Beschleunigungen können filtriert werden. Gleichförmig in beiden Augen wirkende Beschleunigungen, die z.B. in Fahrzeugen auftreten, beeinflussen die Messung des Differenzsignals nicht. Somit ist das künstliche Akkommodationssystem gemäß der Erfindung auch in Straßenbahnen und Aufzügen funktionsfähig. Auch die Bedienung elektrischer Geräte ist ohne Einfluss auf das Akkommodationssystem.

Der Sensor zur Messung der Erdbeschleunigung kann senkrecht im Implantat verbaut werden und ist somit sowohl auf einer Leiterkarte, als auch in einem ASIC integrierbar.
Damit ist eine verbesserte Bauraumausnutzung erzielbar. Die Beschleunigungssensoren zeichnen sich im Vergleich zu Magnetfeldsensoren durch einen reduzierten Energiebedarf aus. Beim Einsatz dieser reduzieren sich auch die für einen Betrieb erforderlichen Energiereserven und damit auch der hierfür erforderliche Platzbedarf im Implantat. Der gewonnene Raum kann z.B. für den Einsatz zusätzlicher Sensoren verwendet werden.

Wird der Neigungswinkel des Kopfes kleiner als 90 Grad, verringert sich der Anteil des Erdbeschleunigungsfeldes in der Sensorebene. Bei exakt horizontalem Sehen verlaufen die Feldlinien senkrecht zur Sensorebene und damit parallel zur Drehachse Z der Augen. Eine Drehung der Augen um diese Achse kann somit nicht erfasst werden und somit auch nicht die Änderung des Vergenzwinkels. Bis zu welchem Winkel zwischen Feld- und Drehachse eine Bestimmung des Vergenzwinkels noch möglich ist, hängt von der Genauigkeit des eingesetzten Sensors ab.

Aus dem Stand der Technik sind Beschleunigungssensoren bekannt (Göpel et al. (Ed.): Sensors, A Comprehensive Survey, Vol. 7: Mechanical Sonors; VCH-Verlag 1994, ISBN 3-527-26773-5, S. 332-358) bei welchen eine statistische Messung ab einem Winkel von ca. 2 Grad zu einem ausreichend genauen Ergebnis führt, auf dessen Grundlage der Akkommodationsbedarf berechnet werden kann. Allerdings ist die Störanfälligkeit für andere Beschleunigungen bis zu einem Winkel von ca. 5 Grad sehr hoch. Im Bereich kleiner als 2 Grad kann der Vergenzwinkel mit Hilfe der Erfassung des Erdbeschleunigungsfeldes nicht bestimmt werden.

Erfindungsgemäß lässt sich dieser Bereich als nicht erfassbar deklarieren. Mit Hilfe der Sensoren zur Messung des Erdbeschleunigungsfeldes wird die Neigung des Kopfes bestimmt und innerhalb des kritischen Neigungswinkels die eingestellte Brechkraft nicht verändert, bis wieder ein verlässliches Signal zur Verfügung steht. Grundsätzlich ist davon auszugehen, dass die augenbezogene Sehachse um ca. 25 bis 30 Grad gegen die Horizontale geneigt ist, wenn der Körper in entspannter Haltung ist. Aus der DIN EN 29 241 ISO 9241-3 ist bekannt, dass der horizontale Blickwinkel im Sitzen bei 20 bis 22 Grad liegt. Das Sehen auf genau horizontaler Ebene wird für den Menschen als anstrengend eingestuft und daher voraussichtlich nur kurzzeitig und mit geringer Häufigkeit eintreten.

In einer Ausführungsform der Erfindung kann die erfindungsgemäße Messung der Erdbeschleunigung in Kombination mit der Messung des Erdmagnetfeldes, wie es in der DE 10 2007 008 374 B4 beschrieben ist (Abschnitte [0034] - [0043] und [0056] sowie [00573]), verwendet werden. D. h. für die horizontale Ausrichtung kommt der Magnetfeldsensor zum Einsatz, während mit der Neigung des Kopfes der Beschleunigungssensor eingesetzt wird. Mit Hilfe des Sensors zur Messsung der Erdbeschleunigung wird bei dieser Anwendung die Neigung des Systems gemessen und damit der Inklinationswinkel bestimmt. Eine weitere Informationsquelle für die Erfassung des Vergenzwinkels auf horizontaler Ebene ist die Messung der Beschleunigungen, die während der Augenrotation auftreten.

Die Drehbewegungen beider Augen werden in Versionen, die gleichsinnige Blickrichtung beider Augen in eine neue Richtung und Vergenz, die gegensinnige Ausrichtung der Augen und damit die Anpassung der Entfernung des Objektes unterteilt. Weitere Bewegungskomponenten in den Augen ergeben sich aus den Kopfbewegungen. Zur Bestimmung des Akkommodationbedarfs ist nur die gegensinnige Vergenzbewegung ausschlaggebend. Sie wird von Version und Kopfbewegung überlagert. Diese Störgrößen sind jedoch in beiden Augen annähernd gleich groß. Sie können somit in der Auswertung kompensiert werden und haben keinen Einfluss auf die Berechnung des Vergenzwinkels. Die Augenbewegungen werden gemessen, indem die Beschleunigungen während der Bewegung erfasst werden. Aufgrund der hohen Geschwindigkeiten sind hierzu sehr hohe Abtastraten des Beschleunigungssensors erforderlich. Anschließend wird mit Hilfe des Vorwissens, wo sich das Auge vor der Bewegung befand und der Information über die Lageänderung, die sich aus der gemessenen Beschleunigung ergibt, die neue Lage errechnet. Da mit dieser Messmethode kein Absolutwert erfasst wird, sondern für die Bestimmung des Vergenzwinkels alle vorangegangen Messungen miteinbezogen werden, addiert sich der Messfehler mit jeder Messung auf. Das System kann mit Hilfe der Messung des Erdbeschleunigungsfeldes bei Neigung des Blickes wieder kalibriert werden, so dass der Einsatz des Magnetfeldsensors für einen begrenzten Zeitbereich möglich ist.

Ein erfindungsgemäß einsehbares Messprinzip von Beschleunigungssensoren ist u.a. die kapazitive Erfassung der Auslenkung einer seismischen Masse. Eine Erfassung über piezoresistive Sensoren ist ebenfalls möglich (Göpel et al. (Ed.): Sensors, A Comprehensive Survey, Vol. 7: Mechanical Sonors; VCH-Verlag 1994, ISBN 3-527-26773-5, S. 332-358). Magnetfelder werden hingegen i.d.R. mit Hilfe des AMR- oder GMR-Effektes (AMR = Anisotroper Magneto Resistiver Effekt, GMR = Giant Magneto Resistiver Effekt) gemessen. Dabei wird die Änderung des Widerstands in Abhängigkeit eines Magnetfeldes erfasst. Für diese Messung ist ein Stromfluss nötig, so dass der Energieverbrauch von Magnetfeldsensoren ca. eine Größenordnung über dem von Beschleunigungssensoren liegt. Auch die Messung der Augenbewegung mit Hilfe von Beschleunigungssensoren verbraucht viel Energie, da hohe Abtastraten für das Erlangen einer ausreichenden Genauigkeit erforderlich sind.

Durch die Kombination von Beschleunigungssensor und Magnetfeldsensor kann die Energiebilanz des erfindungsgemäßen Systems optimiert werden. Die Neigung des Kopfes kann mit Hilfe der Beschleunigungssensoren bestimmt werden. Gelangt das System in die Nähe des für die Erfassung des Erdschwerebeschleunigungsfeldes kritischen Bereichs, wird die zusätzliche Sensoreinheit aktiviert. Anderenfalls wird diese deaktiviert, um Energie zu sparen.

Die mittels der Sensoren erfassten Informationen werden im Rahmen der hier beschriebenen Erfindung dem Informationsverarbeitungssystem zur Verfügung gestellt, in welcher die erfassten Signale aufbereitet werden (z.B. Ausreißertests, Glättung, Filterung, Verstärkung). Mit Methoden der klassischen Statistik, der Computational Intelligence und Data Mining werden Merkmale extrahiert und klassifiziert, um die Akkommodationsabsicht zu detektieren. Mit Hilfe von steuerungs- und regelungstechnischen Methoden (z.B. fuzzy-gesteuerter PID-Regler, adaptive Regelungsalgorithmen, selbstlernende Algorithmen) werden die benötigten Stellsignale für das optische System generiert. Es können sowohl hierarchische Regelungsstrukturen als auch zentral-dezentrale Strukturen zum Einsatz kommen

Zur Versorgung der Subsysteme mit Energie wird ein Energieversorgungssystem eingesetzt, das aus einem Energiewandler, einem Energiespeicher und einer Steuerungseinheit bestehen kann. Der Energiewandler transformiert von außen fernübertragene Energie (z.B. induktiv, kapazitiv, optisch) oder gespeicherte Energie (z.B. Batterie, Miniatur-Brennstoffzelle), die auch in Form von Körperflüssigkeiten (z.B. das nährstoffreiche Kammerwasser, Blut) vorliegen kann, bzw. mechanische Energie (z.B. aus Muskelbewegungen) über einen Energiespeicher in elektrische Energie. Diese wird durch die Steuereinheit des Energieversorgungssystems zu genau definierten Zeitpunkten an die Subsysteme abgegeben. Durch Messung der Beleuchtungsstärke (z.B. durch eine Fotozelle) kann erreicht werden, dass bei Dunkelheit oder bei geschlossenen Augen, d.h. in Situationen, in denen keine Akkommodationsfähigkeit erforderlich ist, das Gesamtsystem in einen Zustand minimalen Energieumsatzes gebracht wird. Die dazu notwendigen Steuersignale werden vom Informationsverarbeitungssystem generiert.

Das Gesamtsystem wird mit Hilfe von geeigneten Befestigungselementen zur axialen Fixierung und radialen Zentrierung im Strahlengang implantiert. Aus der Ophthalmologie (Augenheilkunde) sind für Intraokularlinsen zahlreiche Haptikausführungen bekannt. (Draeger, J.; Guthoff, R.F.: Kunstlinsenimplantation. In: Augenheilkunde in Klinik und Praxis Band 4. Hrsg.: Francois, J.; Hollwich, F. Georg Thieme Verlag Stuttgart New York (1991); Auffarth, G.U.; Apple, D.J.: Zur Entwicklungsgeschichte der Intraokularlinsen. Ophthalmologe 98(11) (2001) 1017-1028). Diese können vorzugsweise im Kammerwinkel, im Sulcus ciliaris oder im Kapselsack Halt finden

Das beschriebene Akkommodationssystem kann zur Wiederherstellung der Akkommodationsfähigkeit nach Entfernung der natürlichen Augenlinse bei Linsentrübung (Katarakt) oder Alterssichtigkeit (Presbyopie) dienen.

Das erfindungsgemäße System ist abgeschlossen und arbeitet autonom. Die Energie muss drahtlos übertragen und im Inneren zwischengespeichert werden. Für die Realisierung auf den begrenzten Bauraum muss darauf geachtet werden, dass die Komponenten selbst sehr klein sind und einen möglichst geringen Energieverbrauch haben, um das benötigte Energiespeichervolumen bei definierter Betriebszeit zu minimieren. Zudem muss eine optimale Nutzung des Bauraums bei der Systemintegration aller Komponenten erfolgen. Das künstliche Akkomodationssystem wird wie eine Intraokularlinse in den Kapselsack implantiert. Aufgrund dessen benötigt es eine Sensoreinheit, die den Akkommodationsbedarf aus dem Inneren des Auges heraus erfassen kann. Mit Hilfe dieser Information kann die aktive Optik in ihrer Brechkraft auf die vom Patienten benötigte Fokuslänge eingestellt werden.

Im Folgenden wird die Erfindung unter Bezugnahme auf die Figuren näher beschrieben:
Fig. 1 zeigt die schematische Darstellung des Gesamtsystem
Fig. 2 zeigt die akkommodative Vergenz
Fig. 3 zeigt die Winkelmessung durch Erfassung der Lage relativ zum Erdbeschleunigungsfeld
Fig. 4 zeigt keine Änderung des messbaren Beschleunigungsfeldes bei horizontaler Blickrichtung
Fig. 5 zeigt die Winkelmessung durch Erfassung der Lage relativ zum Erdmagnetfeld

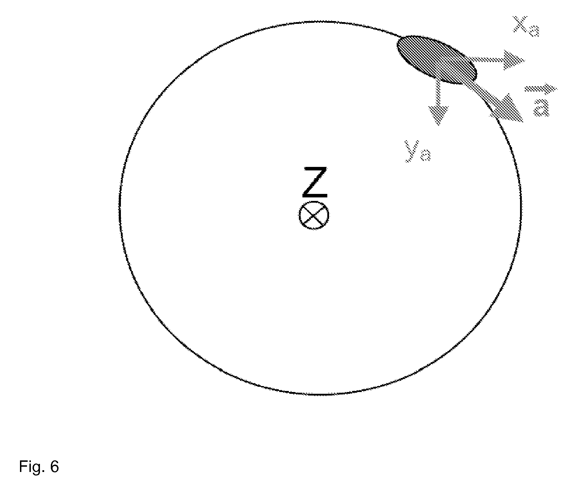

Fig. 6 zeigt die Erfassung der Augenbewegung über Beschleunigungssensoren
Fig. 7 zeigt die Ausrichtung des Magnetfeldsensors im System

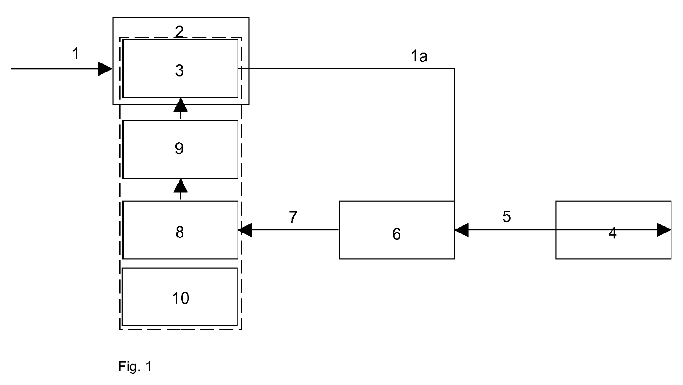

In Figur 1 ist eine schematische Darstellung des Gesamtsystems (künstliches Akkommodationssystem) wiedergegeben. Die Information 1, z.B. Licht von einem Objekt in zeitlich veränderlicher Gegenstandsweite fällt durch den dioptrischen Apparat des menschlichen Auges 2, der das optische System 3 enthält. Das fokussierte Licht 1 a fällt auf den natürlichen Sensor, die Netzhaut 4.

Die durch die Photorezeptoren generierten afferenten Signale 5 werden dem natürlichen Informationsverarbeitungssystem 6, dem Gehirn, zugeleitet. Von dort werden efferente Signale 7, die Information über den Akkommodationsbedarf enthalten, an motorische Strukturen (z.B. Ziliarmuskeln, Bulbusmuskeln) gesendet. Diese Information wird vom Informationserfassungssystem 8 des künstlichen Akkommodations-Systems aufgenommen. Das Informationsverarbeitungssystem 9 leitet daraus Stellsignale für das optische System 3 ab. Damit wird die Scheitelbrechkraft des dioptrischen Apparates 2 durch das künstliche Akkommodationssystem an den Akkommodationsbedarf angepaßt, der aus zeitlich veränderlichen Gegenstandsweiten resultiert. 10 stellt das Energieversorgungssystem dar. Alle technischen Systembestandteile sind durch eine gestrichelte Linie eingerahmt.

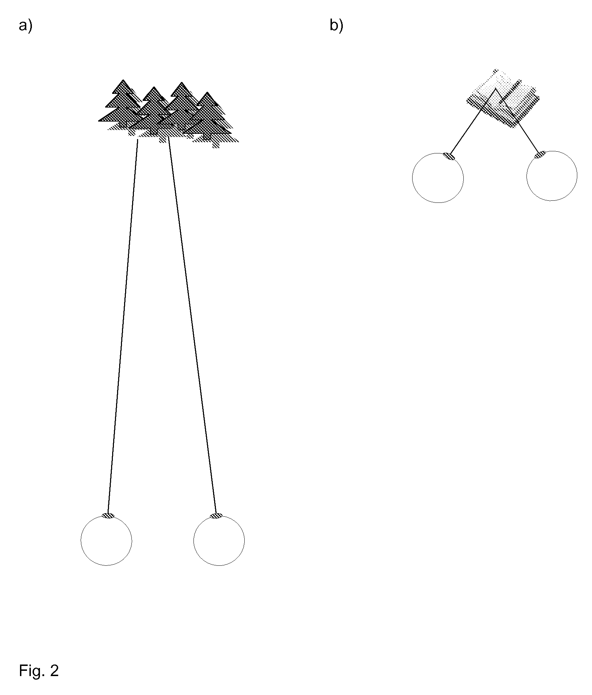

In der Figur 2 ist schematisch die akkomodative Vergenz dargestellt. Hierbei wird in dem Teilbild a) der Zustand bei der Fernsicht und in der Teilansicht b) der Zustand bei der Nahsicht, z.b. beim Lesen, dargestellt. Es ist zu erkennen, dass die akkomodative Vergenz eine gegensinnige Augenbewegung umfasst, die bei der Fixierung von Objekten verschiedener Gegenstandsweiten auftritt. Wird ein Objekt mit großem Abstand (Figur 2 a) betrachtet, verlaufen die Fixierlinien der Augen nahezu parallel. Der Vergenzwinkel ist somit sehr klein. Je näher das Objekt sich relativ zum Betrachter befindet, umso größer wird der Winkel zwischen den Fixierlinien (Figur 2 b). Erfasst man den Vergenzwinkel, kann somit der Akkommodationsbedarf des Menschen ermittelt werden.

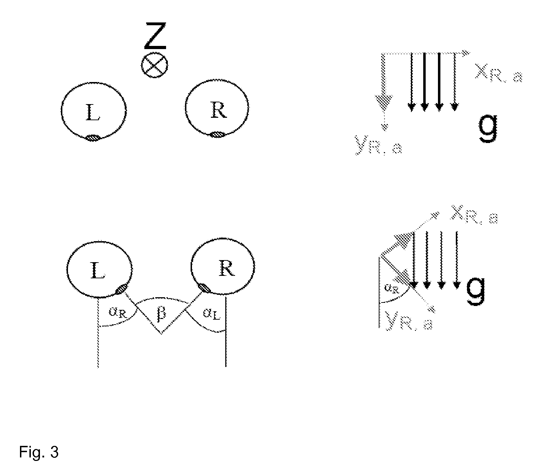

In der Figur 3 ist die Winkelmessung durch Erfassung der Lage der Augen relativ zum Erdbeschleunigungsfeld dargestellt. D.h., um den Vergenzwinkel zu ermitteln, muss die Winkellage der beiden Implantate R und L zueinander ermittelt werden. Die Lage jedes einzelnen Implantates R und L wird relativ zu einer feststehenden Größe gemessen. Als feststehende Größe dient das Beschleunigungsfeld g der Erde.

Gemäß Figur 3 wird von zweiachsigen Sensoren mit den Messachsen x und y ausgegangen. Die Sensorebene x-y ist parallel zum Beschleunigungsfeld der Erde. Die gegensinnige Augenbewegung entspricht einer Drehung um die z-Achse. Der Winkel der Implantatausrichtung im Beschleunigungsfeld g ergibt sich somit aus den gemessenen vektoriellen Anteilen des Beschleunigungsfeldes in x- bzw. y-Richtung des Sensors.

In der Figur 3 ist die Blickrichtung in Richtung des Beschleunigungsfeldes (d.h. senkrecht nach unten) gerichtet. Mit "g" wird das Beschleunigungsfeld bezeichnet. Der Index "a" verweist auf den Beschleunigungsaufnehmer, die Indizes "L" und "R" auf das jeweilige Auge. Bei Fernsichteinstellung in Richtung des Beschleunigungsfeldes (d.h. senkrecht nach unten) bewegen sich die Beschleunigungsfeldlinien parallel zur y-Achse (Fig. 3 obere Hälfte Winkel αL und αR und damit der Vergenzwinkel β = 0). Im Falle einer Nahsicht, z.B. beim Lesen, bilden x und y einen messbaren Winkel α relativ zum Erdbeschleunigungsfeld g. Aus der Messung der x- und y-Anteite kann mithin die Winkel αL und αR und damit der Vergenzwinkel β und damit der Akkommodationsbedarf des Auges berechnet werden.

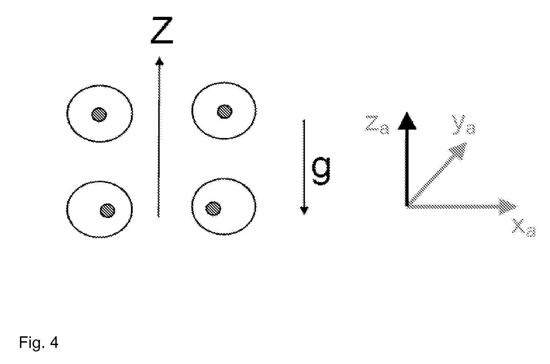

In Figur 4 ist die horizontale Blickrichtung dargestellt. Wird der Neigungswinkel des Kopfes kleiner als 90 Grad, verringert sich der Anteil des Beschleunigungsfeldes in der x-y-Ebene. Bei exakt horizontalem Sehen verlaufen die Feldlinien senkrecht zur Sensorebene und damit parallel zur Drehachse z der Augen. Die Drehung der Augen um die z-Achse kann nicht erfasst werden. Mithin kann auch nicht die Änderung des Vergenzwinkels erfasst werden. Die statische Messung mit Hilfe eines Beschleunigungssensors ist erst ab einem Winkel von ca. 2 Grad möglich. Störanfällig sind die Messungen bis zu einem Winkel von 5 Grad.

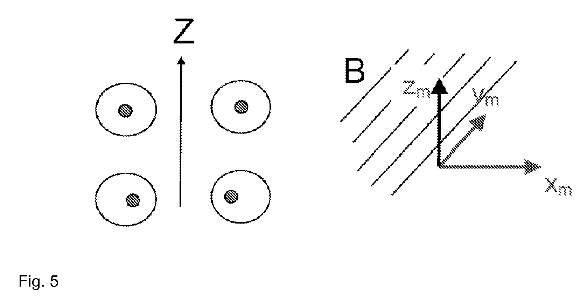

In Figur 5 ist die Winkelmessung durch Erfassung der Lage relativ zum Erdmagnetfeld dargestellt. Im horizontalen Bereich kann hier z.B. durch ein Magnetfeldsensor das Magnetfeld B der Erde erfasst werden und darüber die Ausrichtung eines jeweiligen Auges bestimmt werden. Die Berechnung des Akkommodationsbedarfs erfolgt ebenso wie beim Beschleunigungssensor über die Bestimmung des Vergenzwinkels yₘ:xₘ. Die horizontale Lage ergibt sich aus der Drehachse zₘ der Augen. Der Magnetfeldsensor wird nur im horizontalen Bereich eingesetzt, in dem relativ hohe magnetische Flussdichten detektierbar sind. Hier reicht eine niedrige Auflösung, damit eine kleine Baugröße aus.

In Fig. 5 ist die Erfassung der Augenbewegungen über die Beschleunigungssensoren dargestellt. Die Augenbewegungen können unter Einbeziehung des hochfrequenten Anteils des für die Erdbeschleunigungsmessung eingesetzten translatorischen Beschleunigungssensors erfasst werden. Dieser Anteil wird für die reine Messung des statischen Erdschwerebeschleunigungsfeldes bei niedrigen Abtastraten heraus gefiltert.

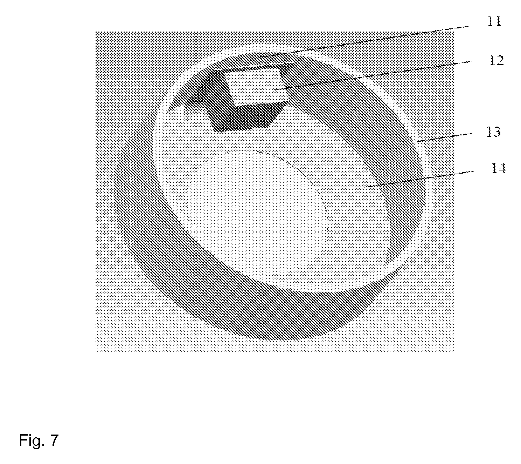

In Figur 7 ist schließlich ein erfindungsgemäß in Kombination mit einem Beschleunigungssensor einsetzbares Magnetfeldsensorsystem dargestellt. Es handelt sich um einen zweiachsigen Sensor, welcher horizontal ausgerichtet ist. In dem Bauraum 11 wird der Magnetsensor 12 eingesetzt. Dieser befindet sich in dem Gehäuse 13. Das Gehäuse 13 umfasst auch die Schaltungsträgerfläche 14. In Fig. 7 wird der schwer nutzbare Bauraum zwischen Gehäuse und Magnetfeldsensor veranschaulicht, der sich bei Einsatz eines Magnetfeldsensors mit horizontaler Ausrichtung grundsätzlich ergibt und mit der Länge des Magnetfeldsensors steigt. Die übrigen Schaltungskomponenten, d.h. die Steuerung, Energieverarbeitung, Beschleunigungssensor, Kommunikationskomponenten können auf einer Fläche des Schaltungsträgers plan zur Stirnfläche des Gehäuses positioniert werden.

## Patentansprüche

1. Implantierbares System zur Bestimmung des Akkommodationsbedarfs in einem künstlichen Akkommodationssystem durch Messung der Augapfelorientierung umfassend
a) wenigstens ein optisches System (3),
b) wenigstens ein Informationserfassungssystem (8) mit Mitteln zur Erfassung der Augapfelorientierung beider Augäpfel als körpereigenes Steuersignal für den Akkommodationsbedarf,
c) wenigstens ein Informationsverarbeitungssystem (9) zwecks Erzeugung eines Stellsignals für das optische System (3) aus den erfassten körpereigenen Steuersignalen
d) wenigstens ein Energieversorgungssystem (10) und
e) wenigstens ein Befestigungssystem,
wobei Übertragungsmittel für den gegenseitigen Informationsaustausch zwischen den Mitteln zur Erfassung der Augapfelorientierung des jeweiligen Augapfels vorgesehen sind,
**dadurch gekennzeichnet, dass** das System in beiden Augen je mindestens ein Mittel zur Erfassung der Augapfelorientierung des jeweiligen Augapfels auf der Basis der Messung des externen Beschleunigungsfeldes aufweist, wobei die Mittel zur Erfassung der Augapfelorientierung fest mit den jeweiligen zugehörigen Augapfel verbunden sind oder in diesen eingesetzt sind.

2. System nach Anspruch 1,
**dadurch gekennzeichnet, dass** es derart ausgestaltet ist, dass es in den Kapselsack des Auges implantierbar ist.

3. System nach einem der vorhergehenden Ansprüche ,
**dadurch gekennzeichnet, dass** es Mittel aufweist, durch welche die Winkellage der Implantate in beiden Augen zueinander gemessen wird.

4. System nach einem der vorhergehenden Ansprüche
**dadurch gekennzeichnet, dass** die Mittel wenigstens zweichachsige Sensoren sind.

5. System nach Anspruch 4,
**dadurch gekennzeichnet, dass** es Sensoren aufweist, die die kapazitive Erfasssung der Auslenkung einer seismischen Masse messen.

6. System nach einem der vorhergehenden Ansprüche,
**dadurch gekennzeichnet, dass** es piezoresistive Sensoren aufweist.

7. System nach einem der vorhergehenden Ansprüche,
**dadurch gekennzeichnet, dass** es Mittel zur Erfassung des Vergenzwinkels und zur Berechnung des Akkommodationsbedarfs enthält.

8. System nach einem der vorgenannten Ansprüche,
**dadurch gekennzeichnet, dass** es Mittel zur Erfassung des hochfrequenten Anteils des für die Erdbeschleunigung eingesetzten translatorischen Beschleunigungssensors aufweist.

9. System nach einem der vorhergehenden Ansprüche
**dadurch gekennzeichnet, dass** es Mittel zur Erfassung des externen Magnetfeldes aufweist.

## Claims

1. An implantable system for determining the accommodation requirement in an artificial accommodation system by measuring the eyeball orientation, comprising
a) at least one optical system (3),
b) at least one data acquisition system (8) which has means for measuring the eyeball orientation of both eyeballs as an endogenous control signal for the accommodation requirement,
c) at least one data processing system (9) for generating an actuating signal for the optical system (3) from the acquired endogenous control signals,
d) at least one energy supply system (10), and
e) at least one fixing system,
wherein the means for transfer for the mutual information exchange are provided between the means for the detection of the eyeball orientation of the respective eyeball
**characterized in that** the system in both eyes has at least one instrument for the detection of the eyeball orientation of the respective eyeball based on the measuring of the external accelerator field, wherein the means for the detection of the eyball orientation are solidly connected with the according respective eyeball or are implemented into it.

2. The system as claimed in claim 1,
**characterized in that** it is designed such that it can be implanted in the capsular sack of the eye.

3. The system as claimed in any of the preceding claims,
**characterized in that** it comprises means by which the angular position of the implants in both eyes to one other can be measured.

4. The system as claimed in any of the preceding claims,
**characterized in that** the means are at least biaxial sensors.

5. The system as claimed in claim 4,
**characterized in that** it has sensors that measure the capacitive detection of the orientation of a seismic mass.

6. The system as claimed in any of the preceding claims,
**characterized in that** it comprises piezoresisitve sensors.

7. The system as claimed in any of the preceding claims,
**characterized in that** it has means for measuring the vergence angle and for the calculation of the accommodation requirement.

8. The system as claimed in one of the preceding claims,
**characterized in that** it has means for the detection of the high frequency component of the translational acceleration sensor used for the gravitational acceleration.

9. The System according to one of the preceding claims **characterized in that** it comprises means for the detection of the external magnetic field.

## Revendications

1. Système implantable servant à déterminer le besoin en accommodation dans un système d'accommodation artificiel par la mesure de l'orientation du globe oculaire comprenant
a) au moins un système optique (3)
b) au moins un système de détection d'informations (8) comportant des moyens de détections de l'orientation des deux globes oculaires en tant que signal de commande endogène pour le besoin en accommodation ,
c)au moins un système de traitement d'information (9) servant à produire un signal de positionnement pour le système optique (3) à partir des signaux de commande endogènes détectés,
d) au moins un système d'alimentation en énergie (10) et
e) au moins un système de fixation,
des moyens de transmission étant prévus pour l'échange d'informations entre les moyens de détection de l'orientation de chaque globe oculaire, **caractérisé en ce que** le système présente, dans les deux yeux, au moins un moyen pour la détection d'orientation du globe oculaire du globe oculaire respectif sur la base de la mesure du champ d'accélération externe, ces moyens de détection de l'orientation de globe oculaire respectif sont solidement liés avec le respectif globe oculaire correspendant ou insérés dans celui-ci.

2. Système selon la revendication 1, **caractérisé en ce**
**que** le système est fait de telle manière que le système est implantable dans le sac de la capsule de l'oeil.

3. Système selon l'une des revendications précédentes, **caractérisé en ce que** que le système a des moyens par lesquels la position de l'angle des implants dans les deux yeux l'un vers l'autre est mesurée.

4. Système selon l'une des revendications précédentes, **caractérisé en ce que** les moyens sont au moins des capteurs à deux essieux.

5. Système selon la revendication 4, **caractérisé en ce que** le système a des capteurs qui peuvent mesurer la capacité du détection de l'orientation d'une masse sismique.

6. Système selon l'une des revendications précédentes , **caractérisé en ce que** le système a des capteurs piézorésistives

7. Système selon l'une des revendications précédentes , **caractérisé en ce que** les système comprend des moyens pour la détection de l'angle de vergence et pour le calcul du besoin de l'accommodation.

8. Système selon l'une des revendications précédentes, **caractérisé en ce que** le système comprend des moyens pour la détection du part de haute fréquence du capteur d'accéleration translationelle usé pour l'accéleration de la gravité.

9. Système selon l'une des revendications précédentes, **caractérisé en ce que** le système a des moyens pour la détection du champ magnétique externe.
